Europäisches Patentamt

European Patent Office

Office européen des brevets

(11) Publication number: **0 668 262 A1**

## EUROPEAN PATENT APPLICATION

(21) Application number: **95100684.0**

(22) Date of filing: **19.01.95**

(51) Int. Cl.⁶: **C07C 67/08**, C07C 69/76

(30) Priority: **18.02.94 JP 21099/94**

(43) Date of publication of application:
**23.08.95 Bulletin 95/34**

(84) Designated Contracting States:
**DE FR GB**

(71) Applicant: **MITSUBISHI GAS CHEMICAL COMPANY, INC.**
**5-2, Marunouchi 2-chome**
**Chiyoda-Ku**
**Tokyo, 100 (JP)**

(72) Inventor: **Uchiyama, Seiji, Mitsubishi Gas Chem. Co., Inc.**
**Niigata Res. Lab.,**
**Shinwari 182,**
**Tayuhama**
**Niigata-shi,**
**Niigata-ken (JP)**

(74) Representative: **Kraus, Walter, Dr. et al**
**Patentanwälte Kraus, Weisert & Partner**
**Thomas-Wimmer-Ring 15**
**D-80539 München (DE)**

(54) **A process for producing dimethyl 4,4'-biphenyldicarboxylate.**

(57) A process for producing dimethyl 4,4'-biphenyldicarboxylate which comprises reacting 4,4'-biphenyldicarboxylic acid and methanol in presence of tin or a tin compound, sufficiently high reaction velocity and yield can be attained by using tin or a tin compound as a catalyst.

BACKGROUND OF THE INVENTION

1) Field of the invention

The present invention relates to a process for producing dimethyl 4,4'-biphenyldicarboxylate which is useful as a highly-functional polyester starting material for fibers, films, resins and the like.

2) Prior Art

In general, dimethyl 4,4'-biphenyldicarboxylate is produced by esterifying 4,4'-biphenyldicarboxylic acid with methanol. However, 4,4'-biphenyldicarboxylic acid cannot be readily dissolved in methanol, so that the reaction velocity is extremely low. It is therfore necessary to carry out the reaction by using an esterification catalyst in order to produce dimethyl 4,4'-biphenyldicarboxylate with industrial advantage.

For instance, the method in which the esterification is carried out in the presence of an acid catalyst such as sulfuric acid as disclosed in Japanese Patent Publication No. 33382/1981 has been conventionally known as a method for esterifying 4,4'-biphenyldicarboxylic acid by the use of an esterification catalyst.

Further, oxides, chlorides and sulfates of zinc, lead, copper, manganese, nickel, antimony, cadmium, cobalt and the like have been conventionally known as catalysts used for the esterification of benzene polycarboxylic acids such as terephthalic acid.

Furthermore, for instance, copper, manganese, rhenium, oxides of copper, oxides of chromium, beryllium sulfate, bismuth sulfate, vanadyl sulfate, tetra-n-butyl titanate, tetra-isopropyl titanate, ferric sulfate, mercury sulfate, zinc sulfate, molybdic acid, phosphonolybdic acid, molybdenum compounds such as oxides and sulfides of molybdenum, basic aluminum acetate, stannous chloride, tellurium dioxide, tungstosilicic acid and the like have been known as catalysts used for the esterification of 2,6-naphthalenedicarboxylic acid.

However, these methods have many drawbacks when viewed from industrial equipment. For example, in the method where sulfuric acid is employed, reactors are required to have resistance to corrosion, so that the cost of productive facilities is increased. In addition, this method is accompanied with the byproduction of dimethyl ether, so that the loss of methanol is great.

The inventors of the present invention tried to apply the above-described known catalysts used for the esterification of benzene polycarboxylic acids such as terephthalic acid, or 2,6-naphthalenedicarboxylic acid to the esterification of 4,4'-biphenyldicarboxylic acid, and found that most of these catalysts are ineffective, and that dimethyl 4,4'-biphenyldicarboxylate is obtained in such an yeild as in the case where no catalyst is used.

From this fact, it was found that even those catalysts which are considered to be useful for the esterification of benzene polycarboxylic acids such as terephthalic acid, or 2,6-naphthalenedicarboxylic acid are not necessarily useful for the esterification reaction between 4,4'-biphenyldicarboxylic acid and methanol.

SUMMARY OF THE INVENTION

The inventors of the present invention made earnest studies on catalysts which can be industrially used with advantage, and, as a result, found that tin and specific tin compounds show specifically excellent catalytic activity in the esterification reaction of 4,4'-biphenyldicarboxylic acid, and has established the present invention.

That is, the present invention provides a process for producing dimethyl 4,4'-biphenyldicarboxylate which comprises: 4,4'-biphenyldicarboxylic acid and methanol are reacted in presence of tin or a tin compound to produce dimethyl 4, 4'-biphenyldicarboxylate.

DETAILED DESCRIPTION OF THE INVENTION

4,4'-Biphenyldicarboxylic acid prepared by any method may be used in the present invention. For instance, 4,4'-biphenyldicarboxylic acid which is obtained by oxidizing 4,4'-dialkylbiphenyl, 4-alkyl-4'-formylbiphenyl or 4,4'-diformylbiphenyl is used.

In the process of the present invention, tin or a tin compound is used as a catalyst for the esterification reaction between 4,4'-biphenyldicarboxylic acid and methanol.

Tin for use as a catalyst in the process of the present invention is more effective when it is used in its powdered sate.

2

A tin compound for use as a catalyst in the process of the present invention is selected from the group consisting of di-n-butyltin oxide, tin oxalate, tin acetate, stannous oxide, tin diphosphate, stannous sulfate, and one or more of these compounds. These tin compounds are applied to the esterification reaction in a powder or solution state, or in a state supported on a carrier.

The ratio of these esterification catalyst of the present invention to 4,4'-biphenyldicarboxylic acid is 0.05-10% by weight, and preferably 0.2-5% by weight. When the amount of the catalyst is less than 0.05% by weight, the effect of the catalyst is very small. On the other hand, even when more than 10% by weight of the catalyst is used, the reaction velocity and yield cannot be further improved, so that such an amount is unfavorable from economic point of view.

The amount of methanol used for the esterification reaction is 1-10 times, and preferably 3-8 times by the weight of 4,4'-biphenyldicarboxylic acid. It is unfavorable to use an excessively small amount of methanol because the esterification reaction proceeds slowly when such an amount of methanol is used.

On the other hand, even when the amount of methanol is made larger than the amount required, the reaction velocity and yield cannot be further improved, and a large amount of unreacted methanol is recovered; such an amount is thus economically disadvantageous.

The reaction temperature of the esterification is 200-350°C, and preferably 230-280°C. When the temperature is lower than 200 °C, the reaction velocity is low, so that it takes many hours to carry out the reaction. On the other hand, it is unfavorable to carry out the reaction at a temperature higher than 350°C because many side reactions such as decomposition, which bring about decrease in yield, are caused at such a high temperature.

The reaction time varies depending upon the amount of the catalyst and the reaction temperature. However, in general, it is approximately 30 minutes to 5 hours. The reaction may be carried out in any of batch-wise, semibatch-wise and continuous manners.

PREFERRED EMBODIMENTS OF THE INVENTION

The present invention will now be explained more specifically by referring to the following Examples and Comparative Examples which are not limitative of the present invention.

Examples 1-12

Predetermined amounts of 4,4'-biphenyldicarboxylic acid, methanol and an esterification catalyst were charged in a reaction vessel of SUS 316 having an internal volume of 100 ml, and reacted with shaking at a predetermined temperature for a predetermined period of time.

After cooling, the content of the vessel was taken into dimethylacetamide and dissolved therein, and then subjected to gas chromatographic analysis. Thus, the yield of dimethyl 4,4'-biphenyldicarboxylate to 4,4'-biphenyldicarboxylic acid was determined. The results are shown in Table 1.

(Table 1)

| Ex. | Catalyst | | Amount Charged | | Temperature(°C) | Time(hr) | Yield (mol%) |
|---|---|---|---|---|---|---|---|
| | Type | Amount Added(g) | BPDA(g) | MeOH(g) | | | |
| 1 | Tin (powder) | 0.12 | 6.0 | 39.7 | 260 | 1.5 | 95.8 |
| 2 | Tin (powder) | 0.15 | 7.5 | 29.8 | 260 | 1.5 | 95.0 |
| 3 | Tin (powder) | 0.04 | 7.5 | 29.8 | 260 | 1.5 | 95.1 |
| 4 | Tin (powder) | 0.04 | 7.5 | 29.8 | 250 | 2.0 | 94.7 |
| 5 | Tin (powder) | 0.15 | 7.5 | 29.8 | 260 | 1.0 | 94.9 |
| 6 | Di-n-butyltin oxide | 0.10 | 5.0 | 39.7 | 250 | 1.5 | 95.7 |
| 7 | Di-n-butyltin oxide | 0.10 | 5.0 | 39.7 | 240 | 1.5 | 91.9 |
| 8 | Stannous oxide | 0.10 | 5.0 | 39.7 | 250 | 1.5 | 96.4 |
| 9 | Tin oxalate | 0.10 | 5.0 | 39.7 | 260 | 1.5 | 97.0 |
| 10 | Tin acetate | 0.10 | 5.0 | 39.7 | 280 | 1.5 | 96.5 |
| 11 | Tin diphosphate | 0.10 | 5.0 | 39.7 | 280 | 1.5 | 97.0 |
| 12 | Stannous sulfate | 0.10 | 5.0 | 39.7 | 280 | 1.5 | 93.3 |

Note

1) BPDA: 4,4'-biphenyldicarboxylic acid

2) MeOH: methanol

Comparative Examples 1-8

Reaction and analysis were conducted in the same manner as in Examples 1-12, and the yield of dimethyl 4,4'-biphenyldicarboxylate to 4,4'-biphenyldicarboxylicacid was determined. The results are shown in Table 2.

(Table 2)

| Comp. Ex. | Catalyst | | Amount Charged | | Temperature(°C) | Time(hr) | Yield (mol%) |
|---|---|---|---|---|---|---|---|
| | Type | Amount Added(g) | BPDA(g) | MeOH(g) | | | |
| 1 | Non-catalyst | - | 5.0 | 39.7 | 280 | 1.5 | 53.2 |
| 2 | Non-catalyst | - | 5.0 | 39.7 | 270 | 2.5 | 25.0 |
| 3 | Molybdenum oxide | 0.10 | 5.0 | 39.7 | 280 | 1.5 | 56.0 |
| 4 | Phosphomolybdic acidg | 0.10 | 5.0 | 39.7 | 260 | 1.5 | 24.9 |
| 5 | Zinc oxide | 0.10 | 5.0 | 39.7 | 260 | 1.5 | 19.2 |
| 6 | Nickel oxide | 0.10 | 5.0 | 39.7 | 260 | 1.5 | 8.7 |
| 7 | Tellurium dioxide | 0.10 | 5.0 | 39.7 | 280 | 1.5 | 58.0 |
| 8 | Cuprous oxide | 0.10 | 5.0 | 39.7 | 260 | 1.5 | 10.6 |

According to the present invention, sufficiently high reaction velocity and yield can be attained by using tin or a tin compound as a catalyst when 4,4'-biphenyldicarboxylic acid and methanol are reacted to produce dimethyl 4,4'-biphenyldicarboxylate.

**Claims**

1. A process for producing dimethyl 4,4'-biphenyldicarboxylate which comprises: 4,4'-biphenyldicarboxylic acid and methanol are reacted in presence of tin or a tin compound.

2. The process according to Claim 1, wherein the tin compound is at least one compound selected from the group consisting of di-n-butyltin oxide, tin oxalate, tin acetate, stannous oxide, tin diphosphate and stannous sulfate.

3. The process according to Claim 1, wherein the amount of the catalyst used is 0.05-10% by weight of the 4,4'-biphenyldicarboxylic acid.

4. The process according to Claim 1, wherein the esterification reaction is carried out at a temperature of 200-350°C.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,Y | EP-A-0 611 746 (MONSANTO COMPANY)<br>* page 3, line 7 - line 24 *<br>* page 4 - page 5; examples I,III *<br>* page 5; table 1 *<br>* page 9 - page 10; claims *<br>--- | 1-4 | C07C67/08<br>C07C69/76 |
| Y | FR-A-2 252 328 (TEIJIN LIMITED)<br>* page 3, line 30 *<br>* page 6, line 2 - line 13 *<br>* page 13; example 77 *<br>* page 18 - page 19; claims *<br>--- | 1-4 | |
| Y | FR-A-1 520 215 (FMC CORPORATION)<br>* page 4, right column, paragraph 4 - page 5, left column, paragraph 1 *<br>* page 6; examples 5,6 *<br>* page 7; claims *<br>----- | 1-4 | |

**TECHNICAL FIELDS SEARCHED** (Int.Cl.6)

C07C

The present search report has been drawn up for all claims

| Place of search | Date of completion of the search | Examiner |
|---|---|---|
| THE HAGUE | 9 May 1995 | Kinzinger, J |